# EUROPEAN PATENT APPLICATION

(11) **EP 0 947 198 A1**
(43) Date of publication of application: **06.10.1999**
(21) Application number: 99200915.9
(22) Date of filing: 24.03.1999
(51) Int. Cl.: A61K 31/62

(54) **Pharmaceutical preparation containing folic acid and acetylsalicylic acid**

(30) Priority: 26.03.1998 NL 1008716
(71) Applicant: PHARMACHEMIE B.V., 2031 GA Haarlem (NL)
(72) Inventor: Visscher, Hendrik, 3941 TH Doorn (NL); Vermeer, Johanna Maria Pieternella, 2161 EP Lisse (NL)
(74) Representative: van der Kloet-Dorleijn, G.W.F.

(57) **Abstract**

The invention relates to a pharmaceutical preparation that is suitable for oral administration, for the treatment and prophylaxis of cardiovascular disorders, comprising folic acid and acetylsalicylic acid, or pharmaceutically acceptable salts or esters, or metabolites thereof, as active constituents and a pharmaceutically acceptable excipient. At least one of the active constituents is preferably provided separately with a coating.

## Description

The invention relates to a pharmaceutical preparation that is suitable for oral administration, for the treatment and prophylaxis of cardiovascular disorders, comprising folic acid and acetylsalicylic acid, or pharmaceutically acceptable salts or esters, or metabolites thereof, as active constituents and a pharmaceutically acceptable excipient.

Such a combination preparation for the treatment of cardiovascular disorders has been unknown until now.

It has been found that the application of both the stated active constituents in the same preparation has considerable advantages, because of their different spectrum of action, and to all appearances may make a lower daily maintenance dosage possible.

It is pointed out that the application of folic acid in a combination preparation is known per se from WO 97/38694. In addition to folic acid this known preparation in every case contains a 3-hydroxy-3-methylglutaryl co-enzyme A (i.e. HMG-CoA) reductase inhibitor. In addition it can optionally contain another agent, such as aspirin (i.e. acetylsalicylic acid) for example. Acetylsalicylic acid is not an HMG-CoA reductase inhibitor, however.

The advantages of a preparation which is exclusively based on folic acid and acetylsalicylic acid as active constituents cannot therefore be deduced from the aforesaid publication.

Folic acid (or folate) is needed in the body for the conversion of homocysteine to methionine, an essential amino acid.

A factor which has been found to be of importance in cardiovascular disorders is the thickening of the wall of the carotid artery ("arteria carotis"). Homocysteine plays a role in this thickening; at present the disorder hyperhomocysteinaemia is therefore associated with atherosclerotic and thrombotic complications.

Folic acid is absorbed in the small intestine. There it is reduced to the active metabolite folinic acid, the active isomer of which is levofolinic acid. Under the influence of dihydrofolic acid reductase (DHFR) this is converted to 5-methyltetrahydrofolic acid and then transported to the tissues. At low dosages this conversion is virtually complete.

It has now been found (see Am.J. Epidemiol., Vol. 143, No. 9, 1996, pp. 845-859) that the plasma folate level is inversely proportional to the plasma homocysteine level; for optimizing the homocysteine levels the recommended folate dosages should be at least 300-400 µg/day.

Homocysteine concentrations of 12.1 µmol/l, or 38.0 µmol/l after addition of methionine, are associated with an increased risk. Serum folate concentrations of 3 ng/ml give a relative risk of 1.7 in comparison with folate concentrations of 6 ng/ml. 0.4 mg of folate per day is able to increase the serum folate concentration to 6.5 ng/ml and the homocysteine concentration to 7.7 µmol/l.

Administration of folic acid is therefore per se an adequate method for combating hyperhomocysteinaemia, and thus reducing the risk of cardiovascular disorders.

As has been stated above, the preparation according to the invention also contains acetylsalicylic acid. It has been found that acetylsalicylic acid inhibits the cyclooxygenase activity of thrombocytes, leading to an inhibition of thrombocyte aggregation. This effect is manifested in a longer bleeding time. Because thrombocytes lack the ability to synthesize new protein, the effect of the loss of cyclooxygenase activity, owing to the definitive nature thereof, can be offset only by the formation of new thrombocytes.

The pharmaceutical preparation according to the invention makes it possible, therefore, to reduce or obviate the risk of cardiovascular disorders, both primary and secondary, via various points of attack.

The preparation according to the invention is preferably a coated or uncoated tablet, capsule, granulate, or solution or dispersion.

In a particular embodiment at least one of the active constituents of the preparation in question is separately provided with a coating, in order to prevent any interaction of folic acid and acetylsalicylic acid. Folic acid can decompose both in an acidic and in an alkaline environment; by separately coating at least one of the active constituents, preferably the folic acid (or folate), such an adverse interaction is obviated and the shelf life of the pharmaceutical preparation is guaranteed over a long period of time.

Ethyl cellulose, hydroxypropyl cellulose, hydroxypropylmethyl cellulose, polymethacrylates and povidone, among other substances, can be used as a coating. Other coating materials can of course also be used, such as for example those for obtaining a slow release, a gastroresistant coating, or an effervescent composition.

When the preparation according to the invention is in the form of a tablet, this can be an ordinary tablet, but also a dispersible, retard, layered, effervescent or enteric-coated tablet, which have compositions known per se to a person skilled in the art.

The pharmaceutical preparation according to the invention is preferably a single dose and contains 0.1 to 7.5 mg of folic acid and 10 to 120 mg of acetylsalicylic acid.

The invention is explained in greater detail by means of the following examples, which are not limiting.

### ORDINARY TABLETS

### Example I

### Tablets for oral use

| Composition | per tablet |
|---|---|
| acetylsalicylic acid | 30.0 mg |
| folic acid | 0.5 mg |
| starch | 12.8 mg |
| lactose | 38.4 mg |
| povidone | 2.5 mg |
| sodium starch glycolate | 5.0 mg |
| microcrystalline cellulose | 10.0 mg |
| silicon dioxide | 0.3 mg |
| magnesium stearate | 0.5 mg |

The mixture is granulated and compacted to tablets with a weight of 100 mg.

The tablets can be provided with a film coating.

The tablets satisfy the disintegration time requirements according to the Ph.Eur. and USP.

### Example II

### Tablets for oral use

| Composition | per tablet |
|---|---|
| acetylsalicylic acid | 30.0 mg |
| folic acid | 5.0 mg |
| starch | 11.7 mg |
| lactose | 35.0 mg |
| povidone | 2.5 mg |
| sodium starch glycolate | 5.0 mg |
| microcrystalline cellulose | 10.0 mg |
| silicon dioxide | 0.3 mg |
| magnesium stearate | 0.5 mg |

The mixture is granulated and compacted to tablets with a weight of 100 mg.

The tablets can be provided with a film coating.

The tablets satisfy the disintegration time requirements according to the Ph.Eur. and USP.

### Example III

### Tablets for oral use

| Composition | per tablet |
|---|---|
| acetylsalicylic acid | 80.0 mg |
| folic acid | 0.5 mg |
| starch | 31.0 mg |
| lactose | 92.8 mg |
| povidone | 6.3 mg |
| sodium starch glycolate | 12.5 mg |
| microcrystalline cellulose | 25.0 mg |
| silicon dioxide | 0.6 mg |
| magnesium stearate | 1.3 mg |

The mixture is granulated and compacted to tablets with a weight of 250 mg.

The tablets can be provided with a film coating.

The tablets satisfy the disintegration time requirements according to the Ph.Eur. and USP.

### Example IV

### Tablets for oral use

| Composition | per tablet |
|---|---|
| acetylsalicylic acid | 80.0 mg |
| folic acid | 5.0 mg |
| starch | 29.8 mg |
| lactose | 89.5 mg |
| povidone | 6.3 mg |
| sodium starch glycolate | 12.5 mg |
| microcrystalline cellulose | 25.0 mg |
| silicon dioxide | 0.6 mg |
| magnesium stearate | 1.3 mg |

The mixture is granulated and compacted to tablets with a weight of 250 mg.

The tablets can be provided with a film coating.

The tablets satisfy the disintegration time requirements according to the Ph.Eur. and USP.

### DISPERSIBLE TABLETS

### Example V

The mixture is compacted to tablets with a weight of 100 mg.

The tablets can be provided with a film coating.

The tablets satisfy the disintegration time requirements according to the Ph.Eur. and USP.

### Example VI

### Tablets for oral use

| Composition | per tablet |
|---|---|
| acetylsalicylic acid | 30.0 mg |
| folic acid | 5.0 mg |
| lactose | 42.7 mg |
| sodium starch glycolate | 3.0 mg |
| microcrystalline cellulose | 12.6 mg |
| starch | 6.7 mg |

The mixture is compacted to tablets with a weight of 100 mg.

The tablets can be provided with a film coating.

The tablets satisfy the disintegration time requirements according to the Ph.Eur. and USP.

### Example VII

### Tablets for oral use

| Composition | per tablet |
|---|---|
| acetylsalicylic acid | 80.0 mg |
| folic acid | 0.5 mg |
| lactose | 115.2 mg |
| sodium starch glycolate | 7.5 mg |
| microcrystalline cellulose | 30.5 mg |
| starch | 16.3 mg |

The mixture is compacted to tablets with a weight of 250 mg.

The tablets can be provided with a film coating.

The tablets satisfy the disintegration time requirements according to the Ph.Eur. and USP.

### Example VIII

### Tablets for oral use

| Composition | per tablet |
|---|---|
| acetylsalicylic acid | 80.0 mg |
| folic acid | 5.0 mg |
| lactose | 110.7 mg |
| sodium starch glycolate | 7.5 mg |
| microcrystalline cellulose | 30.5 mg |
| starch | 16.3 mg |

The mixture is compacted to tablets with a weight of 250 mg.

The tablets can be provided with a film coating.

The tablets satisfy the disintegration time requirements according to the Ph.Eur. and USP.

### RETARD TABLETS

### Example IX

### Tablets for oral use

| Composition | per tablet |
|---|---|
| acetylsalicylic acid | 100.0 mg |
| folic acid | 0.5 mg |
| lactose | 42.4 mg |
| methylhydroxypropyl cellulose | 25.0 mg |
| stearic acid | 6.0 mg |
| silicon dioxide | 0.3 mg |
| magnesium stearate | 0.8 mg |

The mixture is compacted to tablets with a weight of 175 mg.

The tablets form a matrix, as a result of which the release of the active constituents takes place over a longer period.

### Example X

### Tablets for oral use

| Composition | per tablet |
|---|---|
| acetylsalicylic acid | 100.0 mg |
| folic acid | 5.0 mg |
| lactose | 37.9 mg |
| methylhydroxypropyl | |
| cellulose | 25.0 mg |
| stearic acid | 6.0 mg |
| silicon dioxide | 0.3 mg |
| magnesium stearate | 0.8 mg |

The mixture is compacted to tablets with a weight of 175 mg.

The tablets form a matrix, as a result of which the release of the active constituents takes place over a longer period.

### Example XI

### Tablets for oral use

| Composition | per tablet |
|---|---|
| acetylsalicylic acid | 100.0 mg |
| folic acid | 0.5 mg |
| starch | 12.3 mg |
| lactose | 24.5 mg |
| povidone | 6.8 mg |
| calcium hydrogen phosphate | 38.6 mg |
| methacrylic acid copolymer | 26.4 mg |
| acetoglyceride esters | 4.0 mg |
| polysorbate | 1.2 mg |
| talc | 4.0 mg |
| silicon dioxide | 0.6 mg |
| magnesium stearate | 1.1 mg |

The mixture is compacted to tablets with a weight of 220 mg.

The tablets form a matrix, as a result of which the release of the active constituents takes place over a longer period.

### Example XII

### Tablets for oral use

| Composition | per tablet |
|---|---|
| acetylsalicylic acid | 100.0 mg |
| folic acid | 5.0 mg |
| starch | 10.8 mg |
| lactose | 21.5 mg |
| povidone | 6.8 mg |
| calcium hydrogen phosphate | 38.6 mg |
| methacrylic acid copolymer | 26.4 mg |
| acetoglyceride esters | 4.0 mg |
| polysorbate | 1.2 mg |
| talc | 4.0 mg |
| silicon dioxide | 0.6 mg |
| magnesium stearate | 1.1 mg |

The mixture is compacted to tablets with a weight of 220 mg.

The tablets form a matrix, as a result of which the release of the active constituents takes place over a longer period.

### EFFERVESCENT TABLETS

### Example XIII

### Tablets for oral use

| Composition | per tablet |
|---|---|
| acetylsalicylic acid (coated) | 30.0 mg |
| folic acid (coated) | 0.5 mg |
| mannitol | 70.7 mg |
| sodium hydrogen carbonate | 75.0 mg |
| citric acid | 57.5 mg |
| polyethylene glycol | 6.3 mg |
| sodium benzoate | 10.0 mg |

The mixture is compacted to tablets with a weight of 250 mg.

The tablets disintegrate in water releasing carbon dioxide.

The tablets satisfy the disintegration time requirements according to the Ph.Eur. and USP.

### Example XIV

### Tablets for oral use

| Composition | per tablet |
|---|---|
| acetylsalicylic acid (coated) | 30.0 mg |
| folic acid (coated) | 5.0 mg |
| mannitol | 66.2 mg |
| sodium hydrogen carbonate | 75.0 mg |
| citric acid | 57.5 mg |
| polyethylene glycol | 6.3 mg |
| sodium benzoate | 10.0 mg |

The mixture is compacted to tablets with a weight of 250 mg.

The tablets disintegrate in water releasing carbon dioxide.

The tablets satisfy the disintegration time requirements according to the Ph.Eur. and USP.

### Example XV

### Tablets for oral use

| Composition | per tablet |
|---|---|
| acetylsalicylic acid | 80.0 mg |
| folic acid | 0.5 mg |
| mannitol | 142.2 mg |
| sodium hydrogen carbonate | 165.0 mg |
| citric acid | 126.5 mg |
| polyethylene glycol | 13.8 mg |
| sodium benzoate | 22.0 mg |

The mixture is compacted to tablets with a weight of 550 mg.

The tablets disintegrate in water releasing carbon dioxide.

The tablets satisfy the disintegration time requirements according to the Ph.Eur. and USP.

### Example XVI

### Tablets for oral use

| Composition | |
|---|---|
| per tablet | |
| acetylsalicylic acid | 80.0 mg |
| folic acid | 5.0 mg |
| mannitol | 137.7 mg |
| sodium hydrogen carbonate | 165.0 mg |
| citric acid | 126.5 mg |
| polyethylene glycol | 13.8 mg |
| sodium benzoate | 22.0 mg |

The mixture is compacted to tablets with a weight of 550 mg.

The tablets disintegrate in water releasing carbon dioxide.

The tablets satisfy the disintegration time requirements according to the Ph.Eur. and USP.

### LAYERED TABLETS

### Example XVII

### Tablets for oral use

### First layer

| Composition | per tablet |
|---|---|
| acetylsalicylic acid | 30.0 mg |
| starch | 11.7 mg |
| lactose | 40.0 mg |
| povidone | 2.5 mg |
| sodium starch glycolate | 5.0 mg |
| microcrystalline cellulose | 10.0 mg |
| silicon dioxide | 0.3 mg |
| magnesium stearate | 0.5 mg |

The mixture is granulated and compacted to tablets with a weight of 100 mg.

### Second layer

| Composition | per tablet |
|---|---|
| folic acid | 5.0 mg |
| starch | 11.7 mg |
| lactose | 65.0 mg |
| povidone | 2.5 mg |
| sodium starch glycolate microcrystalline | 5.0 mg |
| cellulose | 10.0 mg |
| silicon dioxide | 0.3 mg |
| magnesium stearate | 0.5 mg |

The second mixture is granulated, and compacted around or on the first tablet to a weight of 200 mg.

The tablets can be provided with a film coating.

The tablets satisfy the disintegration time requirements according to the Ph.Eur. and USP.

### Example XVIII

### Tablets for oral use

### First layer

| Composition | per tablet |
|---|---|
| acetylsalicylic acid | 80.0 mg |
| starch | 31.0 mg |
| lactose | 93.3 mg |
| povidone | 6.3 mg |
| sodium starch glycolate | 12.5 mg |
| microcrystalline cellulose | 25.0 mg |
| silicon dioxide | 0.6 mg |
| magnesium stearate | 1.3 mg |

The mixture is granulated, and compacted to tablets with a weight of 250 mg.

### Second layer

| Composition | per tablet |
|---|---|
| folic acid | 0.5 mg |
| starch | 31.0 mg |
| lactose | 172.8 mg |
| povidone | 6.3 mg |
| sodium starch glycolate | 12.5 mg |
| microcrystalline cellulose | 25.0 mg |
| silicon dioxide | 0.6 mg |
| magnesium stearate | 1.3 mg |

The second mixture is granulated, and compacted around or on the first tablet to a weight of 500 mg.
The tablets can be provided with a film coating.
The tablets satisfy the disintegration time requirements according to the Ph.Eur. and USP.

### ENTERIC COATED TABLETS

### Example XIX

### Tablets for oral use

| Composition | per tablet |
|---|---|
| acetylsalicylic acid | 30.0 mg |
| folic acid | 0.5 mg |
| starch | 12.8 mg |
| lactose | 38.4 mg |
| povidone | 2.5 mg |
| sodium starch glycolate | 5.0 mg |
| microcrystalline cellulose | 10.0 mg |
| silicon dioxide | 0.3 mg |
| magnesium stearate | 0.5 mg |

The mixture is compacted to tablets with a weight of 100 mg.

The tablets are then provided with an enteric coating.

The tablets can be provided with a sugar coating.

The tablets satisfy the requirements for gastroresistant tablets according to the Ph.Eur. and USP.

### Example XX

### Tablets for oral use

| Composition | per tablet |
|---|---|
| acetylsalicylic acid | 30.0 mg |
| folic acid | 5.0 mg |
| starch | 11.7 mg |
| lactose | 35.0 mg |
| povidone | 2.5 mg |
| sodium starch glycolate | 5.0 mg |
| microcrystalline cellulose | 10.0 mg |
| silicon dioxide | 0.3 mg |
| magnesium stearate | 0.5 mg |

The mixture is compacted to tablets with a weight of 100 mg.

The tablets are then provided with an enteric coating.

The tablets can be provided with a sugar coating. The tablets satisfy the requirements for gastroresistant tablets according to the Ph.Eur. and USP.

### Example XXI

### Tablets for oral use

| Composition | per tablet |
|---|---|
| acetylsalicylic acid | 80.0 mg |
| folic acid | 0.5 mg |
| starch | 31.0 mg |
| lactose | 92.8 mg |
| povidone | 6.3 mg |
| sodium starch glycolate | 12.5 mg |
| microcrystalline cellulose | 25.0 mg |
| silicon dioxide | 0.6 mg |
| magnesium stearate | 1.3 mg |

The mixture is compacted to tablets with a weight of 250 mg.

The tablets are then provided with an enteric coating.

The tablets can be provided with a sugar coating.
The tablets satisfy the requirements for gastroresistant tablets according to the Ph.Eur. and USP.

### Example XXII

### Tablets for oral use

| Composition | per tablet |
|---|---|
| acetylsalicylic acid | 80.0 mg |
| folic acid | 5.0 mg |
| starch | 29.8 mg |
| lactose | 89.5 mg |
| povidone | 6.3 mg |
| sodium starch glycolate | 12.5 mg |
| microcrystalline cellulose | 25.0 mg |
| silicon dioxide | 0.6 mg |
| magnesium stearate | 1.3 mg |

The mixture is compacted to tablets with a weight of 250 mg.

The tablets are then provided with an enteric coating.

The tablets can be provided with a sugar coating.
The tablets satisfy the requirements for gastroresistant tablets according to the Ph.Eur. and USP.

### CAPSULES/GRANULATE

### Example XXIII

### Capsules or sachets

| Composition | per capsule |
|---|---|
| acetylsalicylic acid (coated) | 30.0 mg |
| folic acid (coated) | 0.5 mg |
| pregelatinized starch | 10.0 mg |
| starch | 20.0 mg |
| lactose | 59.5 mg |

The constituents are mixed and granulated.

Capsules and sachets are each provided with 120 mg of the granulate so formed.

### Example XXIV

### Capsules or sachets

| Composition | per capsule |
|---|---|
| acetylsalicylic acid | 80.0 mg |
| folic acid | 5.0 mg |
| pregelatinized starch | 20.0 mg |
| starch | 40.0 mg |
| lactose | 105.0 mg |

The constituents are mixed and granulated.

Capsules and sachets are each provided with 250 mg of the granulate so formed.

### Example XXV

### Solution for oral use

| Composition | per dose |
|---|---|
| acetylsalicylic acid (as lysine compound) | 30.0 mg |
| folic acid | 0.5 mg |

The mixture is dissolved in a suitable solvent and freeze-dried.

In use it is dissolved in a solvent such as water or a sugar solution.

### Example XXVI

### Solution for oral use

| Composition | per dose |
|---|---|
| acetylsalicylic acid (as lysine compound) | 80.0 mg |
| folic acid | 5.0 mg |

The mixture is dissolved in a suitable solvent and freeze-dried.

In use it is dissolved in a solvent such as water or a sugar solution.

## Claims

1. Pharmaceutical preparation, suitable for oral administration, for the treatment and prophylaxis of cardiovascular disorders, comprising folic acid and acetylsalicylic acid, or pharmaceutically acceptable salts or esters, or metabolites thereof, as active constituents and a pharmaceutically acceptable excipient.

2. Pharmaceutical preparation according to claim 1, wherein the preparation is a coated or uncoated tablet, capsule, granulate, or solution or dispersion.

3. Pharmaceutical preparation according to claim 1 or 2, wherein at least one of the active constituents has been separately provided with a coating.

4. Pharmaceutical preparation according to claims 1-3, wherein the preparation is a unit dose and contains 0.1 to 7.5 mg of folic acid and 10 to 120 mg of acetylsalicylic acid.

5. Use of folic acid and acetylsalicylic acid, or pharmaceutically acceptable salts or esters thereof, for the manufacture of a pharmaceutical suitable for the treatment and prophylaxis of cardiovascular disorders.

6. Use according to claim 5, wherein the pharmaceutical is a unit dose which contains 0.1 to 7.5 mg of folic acid and 10 to 120 mg of acetylsalicylic acid.
